# EUROPEAN PATENT SPECIFICATION

(11) **EP 2 713 962 B1**
(45) Date of publication and mention of the grant of the patent: **20.07.2016**
(21) Application number: 12724477.0
(22) Date of filing: 17.05.2012
(51) Int. Cl.: A61F 2/95

(54) **REDUCED FORESHORTENING STENT WITH BIO-RESORBABLE FIBERS**
STENT MIT BIORESORBIERBAREN FASERN MIT REDZIERTER VERKÜRZUNG
ENDOPROTHÈSE À EFFET DE RACCOURCISSEMENT RÉDUIT AYANT DES FIBRES BIORÉSORBABLES

(30) Priority: 27.05.2011 US 201161491032 P
(43) Date of publication of application: 09.04.2014
(73) Proprietor: Boston Scientific Scimed, Inc., Maple Grove, MN 55311 (US)
(72) Inventor: ANDERSON, James, M., Fridley, MN 55432 (US)
(74) Representative: Vossius & Partner Patentanwälte Rechtsanwälte mbB
(86) International application number: PCT/US2012/038376
(87) International publication number: WO 2012/166380

(56) References cited:
- WO-A2-2008/030488
- US-A- 6 068 634
- US-A1- 2006 047 336
- US-A1- 2007 219 642

## Description

### BACKGROUND OF THE INVENTION

Balloon deployable stents are known in a variety of designs and configurations. During the deployment of certain types of balloon stents, the balloon has a tendency to expand inwardly from the outer ends of the balloon toward the center. This, in turn, opens the outer ends of the stent prior to middle of the stent, which can lead to stent foreshortening.

Another problem encountered when deploying a stent with a balloon occurs when the balloon inflates from one side to the other side and pushes the stent longitudinally along the balloon. This can result in placement of the stent in an undesirable or less desirable location.

Consequently, there remains a need for a balloon stent assembly that reduces foreshortening and promotes proper stent placement.

US 2007/0219642 A1 discloses a stent provided with a series of short pieces or sections connected together by at least one polymer fiber or wire. The polymer fiber or wire can be biodegradable or durable. The fiber polymers may also contain beads, blobs and bulges in the fibers. The stent sections are designed to separate or articulate with time as the body lumen moves in response to biological or physiological events.

The technical problem of the invention is to provide an inflatable balloon and stent assembly facilitating expansion of the stent and avoiding a stent foreshortening.

The problem of the invention is solved by an assembly according to claim 1.

### BRIEF SUMMARY OF THE INVENTION

An inflatable balloon and stent assembly comprises an inflatable balloon and a stent disposed over at least a portion of the inflatable balloon. The stent has a first end portion, a second end portion, and a middle portion therebetween. The balloon and stent assembly further comprises a plurality of bio-resorbable fibers. The bio-resorbable fibers are disposed over at least a portion of the first end portion and the second end portion of the stent.

### BRIEF DESCRIPTION OF THE SEVERAL VIEWS OF THE DRAWING(S)

FIGs. 1A and 1B show a cross-section of a PRIOR ART balloon-stent assembly.
FIG. 2 shows a perspective view of an example of a balloon-stent assembly of the present disclosure.
FIGs. 3A-3C show cross-sectional view of the example of FIG. 2.
FIGs. 4-6 show side views of embodiments of balloon-stent assemblies.

### DETAILED DESCRIPTION OF THE INVENTION

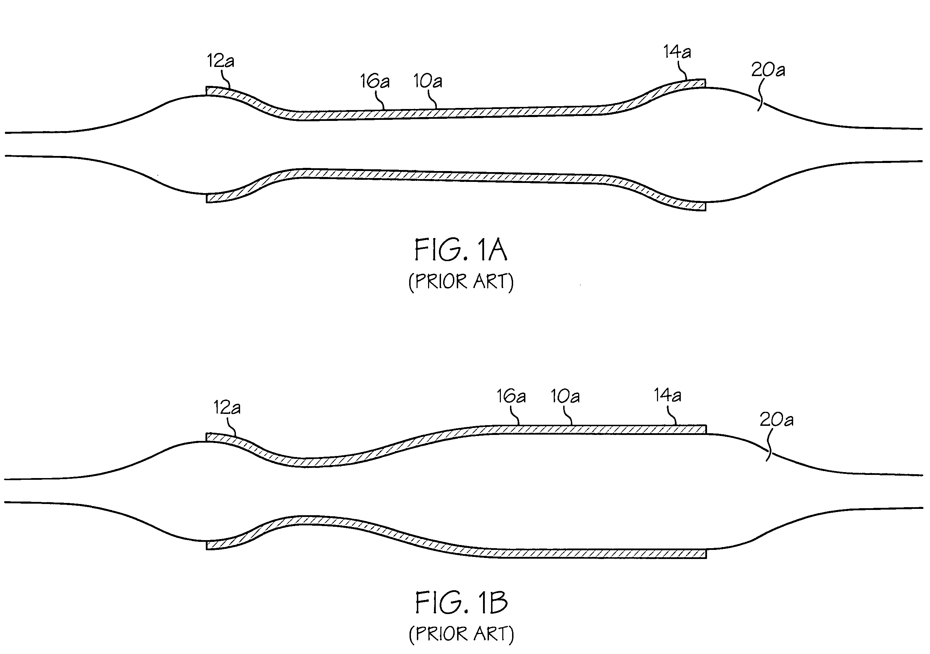

A prior art balloon 20a and stent 10a are shown in cross-section in FIGs. 1A and 1B. FIG. 1A shows the balloon 20a as it begins to expand. Specifically, the balloon 20a is shown expanding at its ends before expanding along its middle. Consequently, the end portions 12a, 14a of the stent 10a expand before the middle portion 16a of the stent 10a.

Turning to FIG. 1B, as the balloon 20a is further expanded, the middle portion 16a of the stent 10a expands, propagating from the expanded end portion 14a to the other expanded end portion 12a. As a result of the end portions 12a, 14a expanding prior to the middle portion 16a, the stent 10a tends to foreshorten.

In addition to the foregoing, stent 10a can also undesirably move axially along the balloon 20a if one end of the balloon 20a inflates before the other end. This can result in improper stent placement.

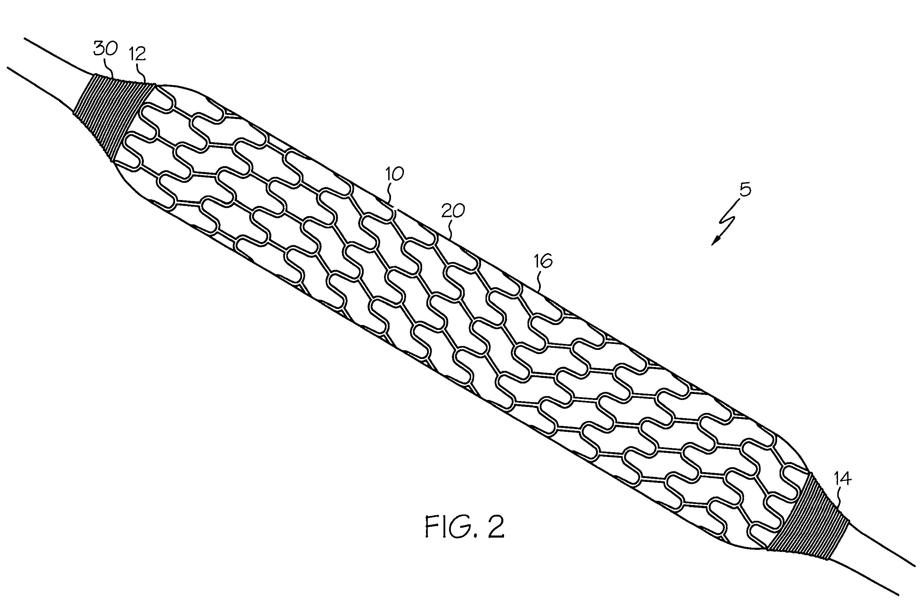

The immediate balloon and stent assembly 5 is designed to overcome these deficiencies and provide for better stent retention on the balloon. In accordance therewith, an inflatable balloon and stent assembly 5 is shown herein in at least one embodiment. As shown for example in FIG. 2, the assembly 5 comprises a stent 10 mounted on an inflatable balloon 20. The stent 10 has a plurality of bio-resorbable fibers 30 disposed around at least a portion of the stent 10. In particular, in some embodiments, the stent 10 has a first end portion 12 and a second end portion 14 around which the bio-resorbable fibers 30 are wound, spun, or otherwise disposed. In some embodiments, the bio-resorbable fibers 30 are located at a first end portion 12 and a second end portion 14 but not a middle portion 16 of the stent 10. The bio-resorbable fibers 30 on the end portions 12, 14 of the stent 10 prevent the end portions 12, 14, from expanding prior to middle portion 16. Thus, in turn, prevents or minimizes foreshortening of the stent 10 and promotes proper stent placement.

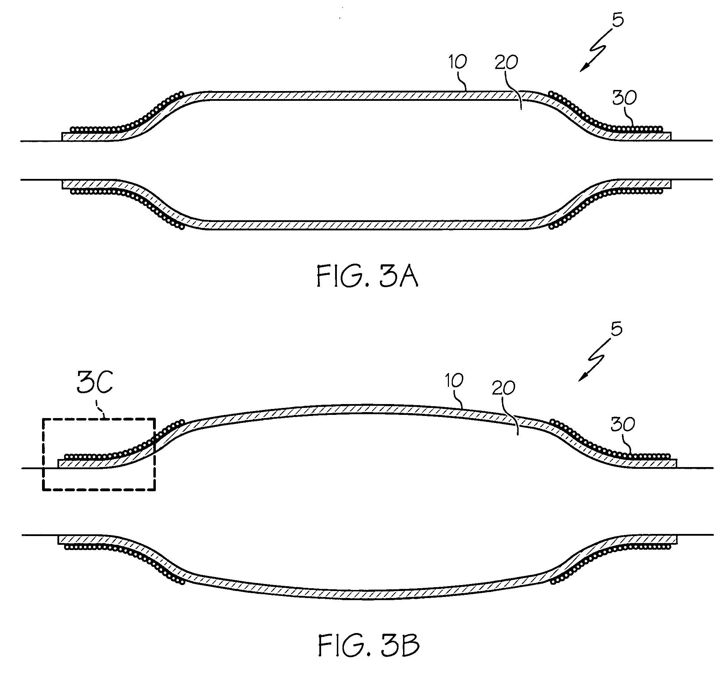

Turning now to FIGs. 3A and 3B, a cross-section of the assembly 5 is shown therein, with stent 10, inflatable balloon 20, and bio-resorbable fibers 30. FIG. 3A shows the assembly 5 as it expands from an unexpanded configuration. For example, the inflatable balloon 20 is expanded to approximately 10 atm, such that the middle portion of the inflatable balloon 20 has expanded but the bio-resorbable fibers 30 restrict expansion of the first and second end portions 12, 14.

As shown in FIG. 3B, as the pressure inside the inflatable balloon 20 is increased, for example to 11 atm, the first and second end portions 12, 14 increase in cross-section and the bio-resorbable fibers 30 expand.

In some embodiments, the bio-resorbable fibers 30 deform plastically upon expansion. In some embodiments, however, the bio-resorbable fibers 30 deform elastically upon expansion. Further, in some embodiments, the bio-resorbable fibers 30 deform elastically upon initial expansion of the balloon 20 and plastically upon further expansion of the balloon 20. In some embodiments, some or all of the bio-resorbable fibers 30 will break upon expansion of the balloon 20 to a desired size.

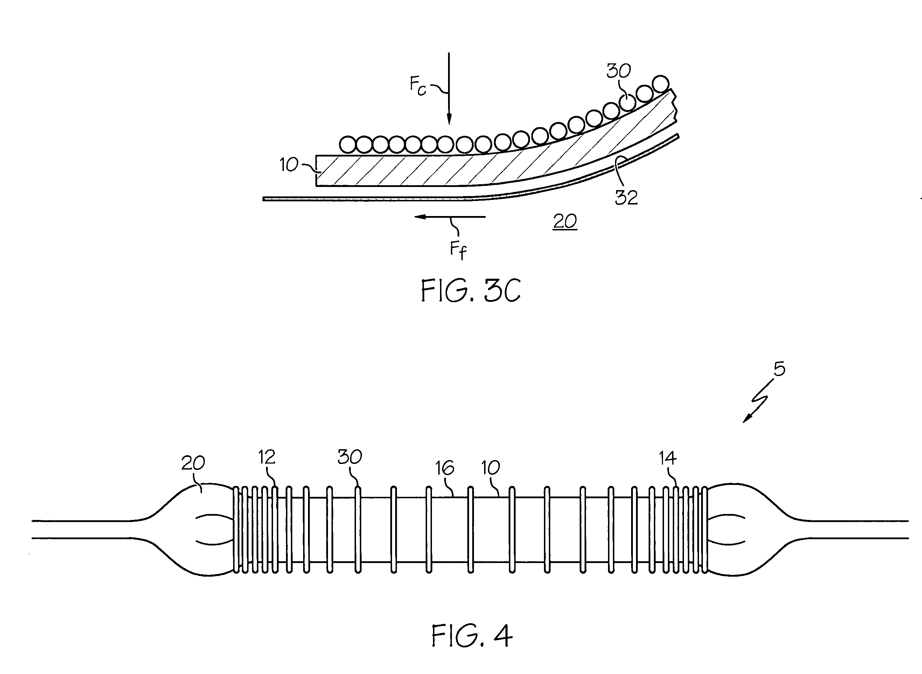

With regard to FIG. 3C, a view of the assembly of FIG. 3B is shown therein. In particular, FIG. 3C depicts the compressive force F_{c} applied to the stent 10 by the bio-resorbable fibers 30. The compressive force F_{c} is applied to at least a portion of the outer surface of the stent 10. The compressive force F_{c} in turn provides a frictional force F_{f} between the stent 10 and the surface 32 of the inflatable balloon 20. The frictional force F_{f} helps to prevent stent foreshortening and axial displacement of the stent 10 relative to the inflatable balloon 20.

As shown in FIGs. 4-6, the stent 10 is crimped on the inflatable balloon 20. Referring specifically to FIG.4, in some embodiments, the bio-resorbable fibers 30 are disposed over portions of the stent 10, including portions of the first and second end portions 12, 14 and the middle portion 16. The number of bio-resorbable fibers 30 per unit length is greater at the end portions 12, 14 than at the middle portion 16. In some embodiments, the number of bio-resorbable fibers 30 per unit length gradually decreases from the end portions 12, 14 of the stent to the middle portion 16. In addition, the bio-resorbable fibers 30 extend onto one or more portions of the inflatable balloon 20 beyond the end portions 12, 14 of the stent 10.

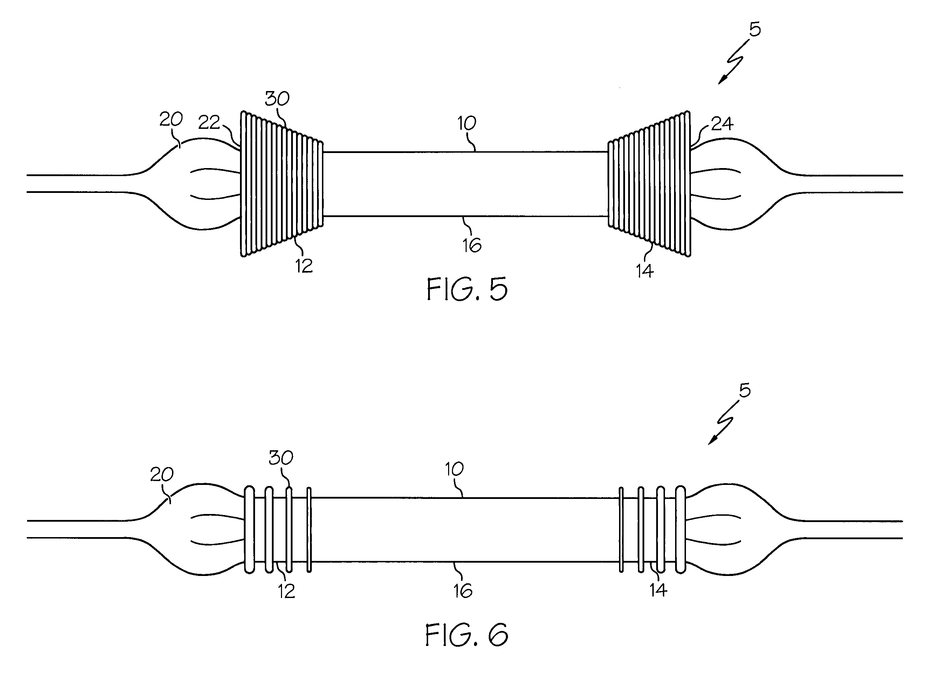

With reference to FIG. 5, in some embodiments, the bio-resorbable fibers 30 are layered. As shown, in some embodiments, the number of layers of bio-resorbable fibers 30 increases nearer the proximal end 22 and distal end 24 of the stent 10. Moreover, the number of layers decreases nearer the middle portion 16 of the stent 10. In some embodiments, the middle portion 16 does not have any bio-resorbable fibers 30 thereon. Alternatively, in some embodiments, the middle portion 16 has bio-resorbable fibers 30 therealong but to a lesser extent than the end portions 12, 14. In this way, bio-resorbable fibers 30 are built up to provide greater resistance to expansion at the end portions 12, 14 and, in some embodiments, at the proximal and distal ends 22, 24.

Turning to FIG. 6, in some embodiments, bio-resorbable fibers 30 at the end portions 12, 14 have a greater diameter than those nearer the middle portion 16. In some embodiments, the diameter of the bio-resorbable fibers 30 decreases longitudinally from the end portions 12, 14 to the middle portion 16. In this way, the stent 10 opens at the middle portion 16 prior to opening at the end portions 12, 14.

In some embodiments, the bio-resorbable fibers 30 are disposed over the stent 10, or portions thereof, by electrospinning. This can be carried out by placing the stent 10 over the inflatable balloon 20 and crimping or otherwise securing the stent 10 to the inflatable balloon 20. Once the stent 10 is in place, the bio-resorbable fibers 30 are added, as desired, to one or more portions of the stent 10 and/or inflatable balloon 20. In some embodiments, the bio-resorbable fibers 30 are electrospun onto the stent 10 and/or inflatable balloon 20 by rotating the assembly 5 while the bio-resorbable fibers 30 are applied. The bio-resorbable fibers 30 are thereby disposed circumferentially around at least a portion of the assembly 5. In some embodiments, the fibers 30 are disposed directly on the outer surface of a metallic stent.

In some embodiments, the bio-resorbable fibers 30 are disposed on the stent 10, or at least portions thereof, prior to the stent 10 being placed on the balloon 20. Alternatively, in some embodiments, bio-resorbable fibers 30 are disposed on the balloon 20, or portions thereof, prior to the stent 10 being placed on the balloon 20. In this way, the bio-absorbable fibers 30 restrict expansion of certain portions of the balloon 20, as desired.

In addition to the particular examples described above, combinations of these examples are also within the scope of this disclosure. For example, in some embodiments, the number of bio-resorbable fibers 30 per unit length is greater at the end portions 12, 14 than at the middle portion 16, for example as shown in FIG. 4 and the diameter of the bio-resorbable fibers 30 decreases longitudinally from the end portions 12, 14 to the middle portion 16, for example as shown in FIG. 6. Other combinations of the aforementioned examples are also contemplated.

In some embodiments, the bio-resorbable fibers 30 are made of polymers, hydro polymers, hydro gels, collagen, or suitable combinations thereof. In some embodiments, the bio-resorbable fibers are made of poly(lactic-co-glycolic acid) (PLGA) or poly(lactic acid) (PLA). In some embodiments, the bio-resorbable fibers 30 are formed from a solution of 7% high molecular weight (HMW) PLGA. In some embodiments, the mixture ratio by weight is 7-10% HMW PLGA/solvent. Further, in some embodiments, the mixture is between 4-40% HMW PLGA by weight.

In some embodiments, for example with lower molecular weight PLA, PLGA copolymers, PLA-b-PEG-b-PLA triblock copolymers, and lactide, were prepared in N, N-dimethyl formamide (DMF) solvent. The concentration of the final solution is, in some embodiments, 30-50% polymer by weight. Other solvents include hexafluoroisopropanol (HFIP) chlorinated solvents, tetrahydrofuran, acetone, or ethyl acetate. Additional additives may include chloroform; N, N-dimethyl formamide (DMF); and 2, 2, 2-trifluoroethanol. Finally, in some embodiments, the solution is of 7% HMW PLGA with HFIP.

In addition, in some embodiments, the ratio of lactide to glycolide in the PLGA is 50:50 (50% lactide and 50% glycolide). Some embodiments employ a 53:47 ratio of lactide to glycolide. Also, in some embodiments, the PLGA includes a lauryl ester group.

In some embodiments, the bio-resorbable fibers 30 are less than 25 microns in diameters, in some embodiments, between 100 nanometers and 25 microns in diameter, and in some embodiments between 200 and 25 microns in diameter. Further, in some embodiments, at least some of the bio-resorbable fibers 30 overlap each other.

Additionally, in some embodiments, the bio-resorbable fibers 30 provide edge protection of the stent 10 during delivery of the stent 10. In particular, in embodiments where the bio-resorbable fibers 30 are disposed over the end 22, 24, of the stent, and particularly the distal end, the bio-resorbable fibers 30 provide a smooth transition from the balloon 20 to the stent 10 as the catheter is being delivered to the patient's vasculature or other organs.

## Claims

1. An inflatable balloon and stent assembly (5) comprising:
an inflatable balloon (20) and a stent (10) disposed over at least a portion of the inflatable balloon (20); the stent having a first end portion (12), a second end portion (14), and a middle portion (16) therebetween; and
a plurality of bio-resorbable fibers (30) disposed over at least a portion of the first end portion (12) and the second end portion (14) of the stent (10), wherein the bio-resorbably fibers (30) further extend onto the surface of the inflatable balloon (20) beyond the end portions (12, 14) of the stent (10) and have a diameter less than 25 microns.

2. The assembly of claim 1, wherein the bio-resorbable fibers are arranged in a circumferential direction.

3. The assembly of claim 1, wherein the stent is crimped on the inflatable balloon.

4. The assembly of claim 1 having an unexpanded configuration and a partially expanded configuration, wherein, in the partially expanded configuration, the middle portion of the balloon is expanded and the first and second end portions are restrained from expanding by the bio-resorbable fibers.

5. The assembly of claim 1, wherein the bio-resorbable fibers have a diameter greater than 100 nanometers.

6. The assembly of claim 1, wherein the bio-resorbable fibers are made of poly(lactic-co-glycolic acid).

7. The assembly of claim 1, wherein the bio-resorbable fibers are further disposed on the middle portion of the stent.

8. The assembly of claim 7, wherein the number of bio-resorbable fibers per unit length is greater at the first and second end portions than at the middle portion.

9. The assembly of claim 8, wherein the first end portion has a proximal end disposed at an end of the stent and the second end portion has a distal end disposed at an end of the stent, the number of bio-resorbable fibers per unit length decreasing from the proximal end to the middle portion and increasing from the middle portion to the distal end.

10. The assembly of claim 7, wherein the first end portion has a proximal end disposed at an end of the stent and the second end portion has a distal end disposed at an end of the stent, and the bio-resorbable fibers are disposed on the first and second end portions of the stent in a plurality of layers, the number of layers decreasing from the proximal end to the middle portion and increasing from the middle portion to the distal end.

## Patentansprüche

1. Anordnung (5) mit einem aufblasbaren Ballon und einem Stent, die aufweist:
einen aufblasbaren Ballon (20) und einen Stent (10), der über mindestens einen Abschnitt des aufblasbaren Ballons (20) angeordnet ist; wobei der Stent einen ersten Endabschnitt (12), einen zweiten Endabschnitt (14) und einen Mittelabschnitt (16) dazwischen aufweist; und
mehrere bioresorbierbare Fasern (30), die über mindestens einen Abschnitt des ersten Endabschnitts (12) und des zweiten Endabschnitts (14) des Stents (10) angeordnet sind, wobei sich die bioresorbierbaren Fasern (30) ferner auf die Oberfläche des aufblasbaren Ballons (20) über die Endabschnitte (12, 14) des Stents (10) hinaus erstrecken und einen Durchmesser von weniger als 25 Mikrometern aufweisen.

2. Anordnung nach Anspruch 1, wobei die bioresorbierbaren Fasern in eine Umfangsrichtung angeordnet sind.

3. Anordnung nach Anspruch 1, wobei der Stent auf den aufblasbaren Ballon gecrimpt ist.

4. Anordnung nach Anspruch 1, die eine nicht expandierte Konfiguration und eine teilweise expandierte Konfiguration aufweist, wobei in der teilweise expandierten Konfiguration der Mittelabschnitt des Ballons expandiert ist und der erste und zweite Endabschnitt durch die bioresorbierbaren Fasern daran gehindert werden, sich zu expandieren.

5. Anordnung nach Anspruch 1, wobei die bioresorbierbaren Fasern einen Durchmesser von mehr als 100 Nanometern aufweisen.

6. Anordnung nach Anspruch 1, wobei die bioresorbierbaren Fasern aus Milchsäure-Glycolsäure-Copolymer bestehen.

7. Anordnung nach Anspruch 1, wobei die bioresorbierbaren Fasern ferner auf dem Mittelabschnitt des Stents angeordnet sind.

8. Anordnung nach Anspruch 7, wobei die Anzahl der bioresorbierbaren Fasern pro Längeneinheit am ersten und zweiten Endabschnitt größer als am Mittelabschnitt ist.

9. Anordnung nach Anspruch 8, wobei der erste Endabschnitt ein proximales Ende aufweist, das an einem Ende des Stents angeordnet ist, und der zweite Endabschnitt ein distales Ende aufweist, das an einem Ende des Stents angeordnet ist, wobei die Anzahl der bioresorbierbaren Fasern pro Längeneinheit vom proximalen Ende zum Mittelabschnitt abnimmt und vom Mittelabschnitt zum distalen Ende zunimmt.

10. Anordnung nach Anspruch 7, wobei der erste Endabschnitt ein proximales Ende aufweist, das an einem Ende des Stents angeordnet ist, und der zweite Endabschnitt ein distales Ende aufweist, das an einem Ende des Stents angeordnet ist, und die bioresorbierbaren Fasern am ersten und zweiten Endabschnitt des Stents in mehreren Schichten angeordnet sind, wobei die Anzahl der Schichten vom proximalen Ende zum Mittelabschnitt abnimmt und vom Mittelabschnitt zum distalen Ende zunimmt.

## Revendications

1. Dispositif à ballonnet gonflable et stent (5), comprenant :
un ballonnet gonflable (20) et un stent (10) disposé sur au moins une partie du ballonnet gonflable (20) ; le stent présentant une première section d'extrémité (12), une deuxième section d'extrémité (14) et une section centrale (16) entre celles-ci ; et
une pluralité de fibres biorésorbables (30) disposées sur au moins une partie de la première section d'extrémité (12) et de la deuxième section d'extrémité (14) du stent (10), lesdites fibres biorésorbables (30) s'étendant en outre sur la surface du ballonnet gonflable (20) au-delà des sections d'extrémité (12, 14) du stent (10), et ayant un diamètre inférieur à 25 microns.

2. Dispositif selon la revendication 1, où les fibres biorésorbables sont disposées dans une direction circonférentielle.

3. Dispositif selon la revendication 1, où le stent est serti sur le ballonnet gonflable.

4. Dispositif selon la revendication 1, présentant une configuration de non-expansion et une configuration d'expansion partielle, où, en configuration d'expansion partielle, la section centrale du ballonnet est expansée et l'expansion de la première et de la deuxième sections d'extrémité est empêchée par les fibres biorésorbables.

5. Dispositif selon la revendication 1, où les fibres biorésorbables ont un diamètre supérieur à 100 nanomètres.

6. Dispositif selon la revendication 1, où les fibres biorésorbables sont en acide poly(lactique-co-glycolique).

7. Dispositif selon la revendication 1, où les fibres biorésorbables sont en outre disposées sur la section centrale du stent.

8. Dispositif selon la revendication 7, où le nombre de fibres biorésorbables par unité de longueur est supérieur sur la première et la deuxième section d'extrémités par rapport à la section centrale.

9. Dispositif selon la revendication 8, où la première section d'extrémité présente une extrémité proximale disposée à une extrémité du stent, et où la deuxième section d'extrémité présente une extrémité distale disposée à une extrémité du stent, le nombre de fibres biorésorbables par unité de longueur diminuant de l'extrémité proximale à la section centrale et augmentant de la section centrale à l'extrémité distale.

10. Dispositif selon la revendication 7, où la première section d'extrémité présente une extrémité proximale disposée à une extrémité du stent, où la deuxième section d'extrémité présente une extrémité distale disposée à une extrémité du stent, et où les fibres biorésorbables sont disposées sur la première et la deuxième sections d'extrémité du stent dans une pluralité de couches, le nombre de couches diminuant de l'extrémité proximale à la section centrale et augmentant de la section centrale à l'extrémité distale.
